# EUROPEAN PATENT SPECIFICATION

(11) **EP 3 104 739 B1**
(45) Date of publication and mention of the grant of the patent: **27.06.2018**
(21) Application number: 14711320.3
(22) Date of filing: 12.02.2014
(51) Int. Cl.: A45D 26/00

(54) **EPILATOR WITH SEPARABLE PAIR OF HEADS**
EPILATIONSVORRICHTUNG MIT ABNEHMBAREM PAAR VON KÖPFEN
ÉPILATEUR COMPORTANT UNE PAIRE DE TÊTES SÉPARABLES

(43) Date of publication of application: 21.12.2016
(73) Proprietor: Epilady 2000 LLC, Hazor Haglilit 10351 (IL)
(72) Inventor: PORAN, Yehuda, Hazor Haglilit 10351 (IL)
(74) Representative: Stellbrink & Partner Patentanwälte mbB
(86) International application number: PCT/IL2014/000013
(87) International publication number: WO 2015/121851

(56) References cited:
- WO-A1-2012/131416
- WO-A1-2013/113423
- US-A1- 2001 014 809

## Description

### Field of the invention

The invention is directed to an epilator and two heads therefor. The heads are provided for trapping hair for removal from a human's body. The heads can be separated from each other from a common support structure or and a support structure for both or each of them.

### Background

In the art a number of epilator and head assemblies are known.

In WO 2009/010815 A1 an epilator is described comprising a head for trapping hair for removal. The head comprises a rotating body that has as an axis of rotation and is adapted to be rotated by a motor. At least one pair of pincers is arranged at or in the rotating body. The pair of pincers is able to rotate in accordance with the rotation of the rotating body. At least their outermost ends are adapted to move towards each other and away from each other depending on the location of the rotating body. According to an aspect, at least one intermediate member is provided which is arranged such that it is at least linearly moveable between the outermost ends of the pincers. The pair of pincers only extends on one side of the axis of rotation. One spring is located between the pair of pincers and is arranged to bias the pincers either away from each other or towards each other. At least one actuator is provided for the pair of pincers which can be supported to move along or parallel to the axis of rotation and engages a first pincer adjacent an end thereof being distal to the axis of rotation and a second pincer at or adjacent an end thereof being proximal to the axis of rotation.

The epilating device according to EP 0 950 365 B1 includes a single housing that is shaped to be grasped by the hand of a user and which includes a drive source. The housing can carry a first epilation module provided at its top with a first plucking head that is driven by the drive source to effect the plucking of the body hairs. The first plucking head has a length defining there along a first epilation zone. Also provided is a second epilation module to be carried on the housing that is provided at its top with a second plucking head that is driven commonly by the drive source to effect plucking of the body hairs. The second plucking head has a length defining there along a second epilation zone.

EP 1 405 701 B1 relates to a linkage mechanism for a hair removal appliance, such as a powered or dry shaver or epilator, having a head rockably mounted on a body, and to such hair depilation apparatus. In order to better conform the head to a user's skin the device and method realizes a rocking about a virtual pivot axis.

WO 2013/113423 A1 relates to a hair remover comprising a frame and two parallel, separate epilation heads with individual suspension for each head, wherein the hair remover also comprises an individual motor for each head. There is still a need for a new concept for a more effective and/or comfortable way of epilating hair.

### Summary of the Invention

It is the object of the present invention to provide a more effective, comfortable, flexible and/or accurate epilator and respective head assembly and respective epilation methods.
The solution of the problem is attained by the subject matter of the claims.
In one aspect the present is directed to pair of corresponding epilator heads for trapping hair for removal, each head comprising a coupling portion for coupling the head to a driving or holding unit. According to an alternative aspect each head is attached to a driving or holding unit in a fixed or releasable manner. In the latter case these driving or holding units can be coupled so as to form an epilator consisting of two heads with two driving units.
The coupling portion preferably comprises positively locking and corresponding elements at the heads and the driving unit. The driving unit preferably embraces a body with one or preferably two motors for each head one. Further compartments for batteries, a socket for a cable and/or an AC/DC transformer can be provided. Preferably the driving unit is designed being able to be easily and safely held by a user.
According to the alternative aspect with each head being coupled to a driving unit, the respective driving units can be attached to each other. This is preferably possible by a set of engaging sliding tracks. In addition or alternatively a release button can be arranged. In this case the sliding tracks are arranged to finally snap into a final position in order to form the completed epilator. The snapping arrangement can be released by the release button that is preferably arranged on or in one of the driving units. Further a contact can be arranged between the two driving units so as to allow a switch for activating the epilator to be arranged on one of the driving units but for controlling both driving units.
Further an exposed portion for allowing at least one or more parts of each head to get in touch with a user's skin is provided. This is preferably realized by a casing with an open window for exposing an epilating tool. This tool is a rotating body having an axis of rotation and is adapted to be rotated by the driving unit or the motors contained therein. The rotating body is adapted to trap the hair for removal at the exposed portion. One preferred arrangement is disclosed in WO 2009/010815 A1. The axis of the rotating body is essentially concave relative to the exposed portion of each head. That is, the rotating body is accessible through the window in the casing of each head. Further pincers are provided being arranged and adapted to grab and epilate hair of a user.
According to another aspect of the invention the rotating body of each pair of epilator heads are positioned side by side when coupled to the driving unit(s).
According to a further alternative aspect the epilator head is adapted to be coupled to the driving unit in a spring-biased fashion so as to allow a spring-biased movement of each epilator head upon pressure thereon during use. The springs are adapted to allow during use a proper displacement depending on the pressure in order to appropriately conform each head to the contours of a user.

In a further aspect of the invention the rotating bodies are adapted to be driven by the driving unit so as to rotate towards each other at the exposed portions. That is, they have an opposing orientation of rotation.

Each coupling portion comprises a coupling element for a corresponding driving rod connected to the driving unit. The driving rod preferably extends out of the driving unit and couples to a head in the connected position. For each head a separate driving rod is provided. The coupling element is preferably provided with an open polygonal recess that is further preferably hexagonal so as to allow an axial relative movement of the driving rod during driving or use. Any other positively locking and easily insertable shape of the recess and the driving rod can also be used in order to make the rotating body revolve or turn.

The coupling element is preferably also provided with gear teeth intermeshing with a larger gear driving the rotating body for reducing its rotating speed and enhancing its torque respectively. The gear teeth are preferably imprinted or casted to conform to an outer contour of coupling element.

According to a further aspect of the invention each head has a snap on mechanism for preferably releasable attaching the head to the driving unit. Such snap on mechanism preferable allows for the spring-biased displacement of each head as described before and below.

Each head preferably having at least one, preferably a pair of springs biasing each head and thereby removing any play and allowing a retraction or displacement during use of so as to comfortably adapt to the contours of a user upon sufficient pressure.

Each head is preferably adapted to be snapped onto the driving unit of the epilator by a tooth-like snap protruding from the drive unit into a respectively undercut edge or vice versa. As mentioned before a retracting movement or displacement during use should preferably be possible.

As mentioned before, one aspect of the invention can implement rotating bodies with pincers as disclosed by WO 2009/010815 A1. In more detail each head is in this case provided with at least two pincers. The pincers comprise at least one substantially radially extending arm wherein the arm is provided with a clamping end. Moreover an engager engages the pincers and drives the pincers in use. A driven actuator driven causes a relative movement of at least a portion of the pincers so as to make the pincers trapping hair during use of the device. At least one spacer is located between the two pincers, the spacer being preferably being made of rubber and being more preferably a standard rubber ring as known for sealing purposes. This is an advantage as preferably a standard element can be used. An axis further receives the pincers, the spacer and/or at least two adjacent discs. The discs further comprise a hub that axially protrudes from the center of a first side of the disc wherein the hub receives the pincers and the spacer. At least one first protrusion substantially axially extends from the first side of the disc and is adapted to cause the pincers which are received by the adjacent disc to trap hair during use.

According to a further aspect of the present invention a whole device or epilator is also covered. All aspects and features mentioned before and below are also contained in such epilator.

The epilator further comprises a driving unit with a motor, preferably for each head. This makes it possible to make use of two smaller devices and to avoid making use of a stronger motor driving both heads by further gear elements. The revolutions of the heads don't need to be exactly adapted or conformed to each other.

The drive unit preferably has a driving pin for each head. Such driving pin directly or indirectly extends from each motor and extends into a respective coupling element of each head. Each driving pin is preferably at its outer end provided with a positively locking contour of hexagonal shape corresponding to the one of the respective coupling element. This is preferably a male / female connecting arrangement and doesn't need to be exact in its length. Further preferably the driving pins and engaging coupling elements are adapted such that a gap is provided between the ends of the driving pins and the bottoms of the coupling elements. This play makes it possible to compensate for changes in distance between the heads and the drive unit during use, particularly when the heads are displaced towards the driving unit by the pressure being applied onto them during use.

In a further aspect the present invention is also directed to an epilation method making use of the before and below described aspects and features discussed in connection with the heads and / or the epilator. Such method can concern the operating of a pair of corresponding epilator heads for trapping hair for removal and comprises the following steps of coupling each head to a common driving unit, adapting at least one or more parts of each head being able to get in touch with a user's skin by an exposed portion, revolving in each head a rotating body having an axis of rotation and being rotated by the driving unit thereby causing the rotating bodies to trap the hair for removal at the respective exposed portion and wherein the axis of each rotating body is essentially concave relative to the exposed portion of each head.

Instead of or additionally to the latter feature the step of coupling the epilator head to the driving unit in a spring-biased fashion is provided according to another aspect of the invention so as to allow a spring-biased movement of each epilator head upon pressure thereon during use.

### Preferred embodiments

The figures attached exemplify the present invention and aspects thereof. They are not intended to limit the invention to the specific embodiments shown.

The figures show the following
Fig. 1 an assembled epilator according to a preferred aspect of the present invention;
Fig. 2 an exploded view with preferred aspects of the head assemblies, a supporting structure and motors for driving the epilator;
Fig. 3 an bottom view of a preferred aspect of head assemblies according the present invention;
Fig. 4 detailed perspectives and insights onto or into preferred aspects of head assemblies and a support structure according to the present invention;
Fig. 5 a perspective view onto another preferred aspect of the present invention with an completed epilator comprising two separable head assemblies each attached to a corresponding supporting unit;
Fig. 6 a first perspective view onto the preferred aspect of the present invention according to Fig. 5 with two separated head assemblies each attached to a corresponding supporting unit; and
Fig. 7 a second perspective view onto the preferred aspect of the present invention according to Fig. 5 and Fig. 6 with two separated head assemblies each attached to a corresponding supporting unit.

### Detailed description

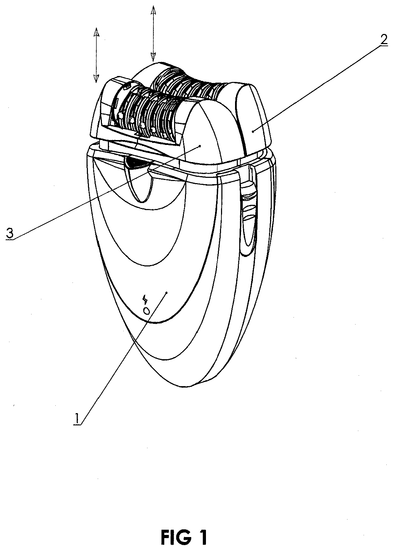

According to Fig. 1 a view onto a preferred aspect of the present invention is shown. A driving unit 1 with a body can comprise one or more batteries and/or a socket for a cable for loading the device or for supplying power to one or more motors (not shown) contained in the driving unit 1. Onto the driving unit 1 a rear or first head 2 and a front or second head 3 is shown. These heads 2,3 are mounted onto the driving unit 1, preferably in a manner allowing some movement up and down as indicated by the arrows. This movement is biased as to essentially ensure that the heads are in the outermost positions before use. When during use pressure is exerted onto the heads by attaching and slightly pressing the device against a user's skin they can preferably more downwardly in order to better conform to the contours of the user's skin under treatment.

Moreover a knob is shown on the right hand side and also present on the opposite side in order to allow one or both heads 2,3 to be released from the driving unit 1. Any other arrangement can fulfill the same function.

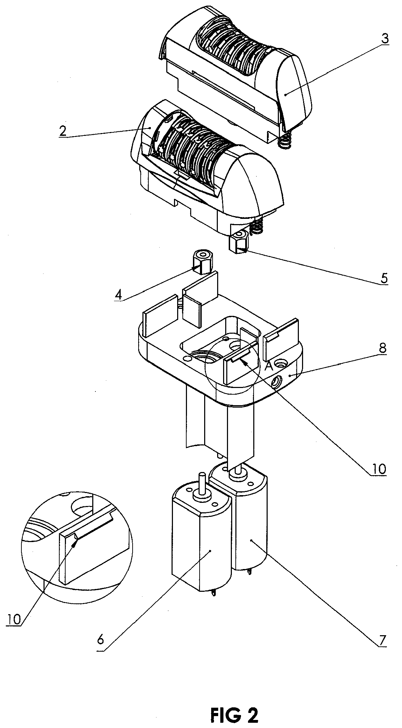

According to Fig. 2 an exploded drawing shows the heads 2,3 in a released position. Below are shown elements that are preferably assembled to the driving unit 1. One motor 6,7 is shown for driving each head 2,3. The driving pin extends in this embodiment to the upper side towards the heads 2,3. On top of such driving pins is a polygonal element such as a hexagon 4,5 in order to engage the respective coupling element (not shown) to each head 2,3. A support 8 is also shown for supporting the motors 6,7. In the assembled status the driving rods of the motors 6,7 extend through the support 8 and the hexagons 4,5 are placed on top of them.

A snap on element or latch 10 is also shown which in this preferred embodiment is provided on a tongue of support 8. Preferably 4 latches are provided, a pair for each head 2,3. Such latches engage respective elements (not shown) provided in the heads 2,3. Such latches can be brought out of engagement by the respective knobs.

The rotating bodies in heads 2,3 are covered at their ends by the head cover and allow the accessibility of them to the user's skin in use through respectively formed windows.

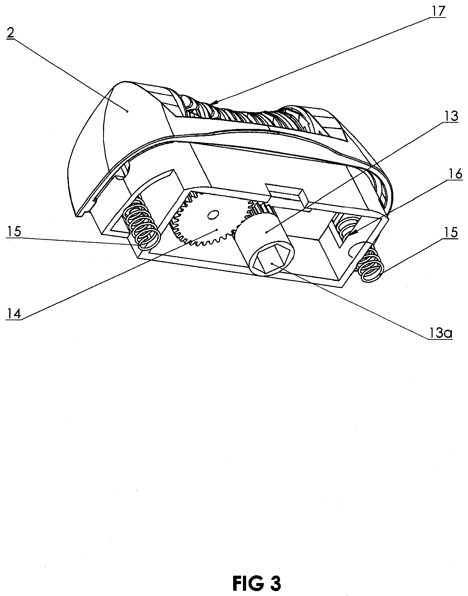

Fig. 3 depicts an example of a head 2 from the underside that is directed towards or onto the driving unit (not shown) in use. As already also visible in the previous Fig. 2 the concave shape of the rotating body 17 towards the user in use is apparent.

Moreover, a coupling 13 with a coupling element 13a can be seen which usually engages the driving pin of the motor (not shown) in use. As mentioned this connection between the coupling element 13a and the driving pin allows for some movement of the driving pins within the coupling elements 13a in uses, particularly for compensating the displacement of the respective head in use.

At the bottom of the coupling element 13a gear teeth are visible forming a gear. This intermeshes with another gear 14 for preferably reducing the turning speed or respectively enhancing the torque of the respective rotating body 17. The gear 14 is further connected with gears (not shown) driving the rotating body 17.

Moreover, a pair of springs 15 is shown biasing the head 2 against the support as shown in the previous figure. They cause the biasing and the possibility of displacement of the head during use when sufficiently pressed to the skin of a user.

A window 16 is also apparent in Fig. 3 for allowing the latches (not shown) provided in the support to engage and to releasably hold the head 2 in the outermost position when no displacement takes place. This is usually the case when the head 2 is attached to the driving unit but when the device is not in use or no pressure is exerted onto the head 2.

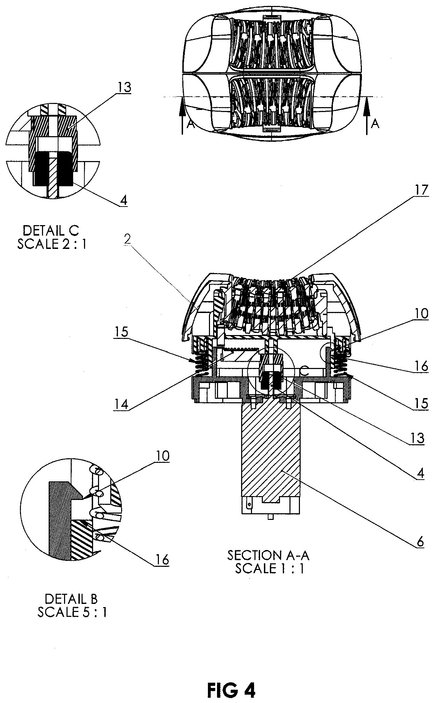

Fig. 4 shows in the upper part a top view onto the two heads with the exposed rotating bodies being visible through the windows of the head's casing. The hair of a user preferably is clamped or grasped and pulled into the gap between the two rotating bodies and thereby epilated or removed.

Further below is shown a sectional view A-A onto the support, one motor 6 and one head 2. The exposed part of the rotating body 17 is shown in its concave configuration from the side in order to better conform to the skin of a user. The front hexagon 5 is shown in the engagement with the coupling 13 and the gear formed therein. Also the dimensions and tolerances allowing the displacement of the head 2 downwardly or towards the driving unit (not shown) are apparent. This is even better shown in Detail C showing hexagon 6 engaging coupling 13.

During the activation of the motor 6 the coupling 13 is turned which drives the further gear 14 that itself drives another gear within the head 2 for driving or making the rotating body 17 turn.

In section A-A the functioning of the head 2 and its biasing arrangement onto the support or driving unit by springs 15 is already apparent. Further the engagement of the latch 10 into the window 16 is shown. This part is shown enlarged in Detail B showing the displacement of the head towards the driving unit (not shown) or the support by the distance of latch 10 and the lowermost edge of window 16.

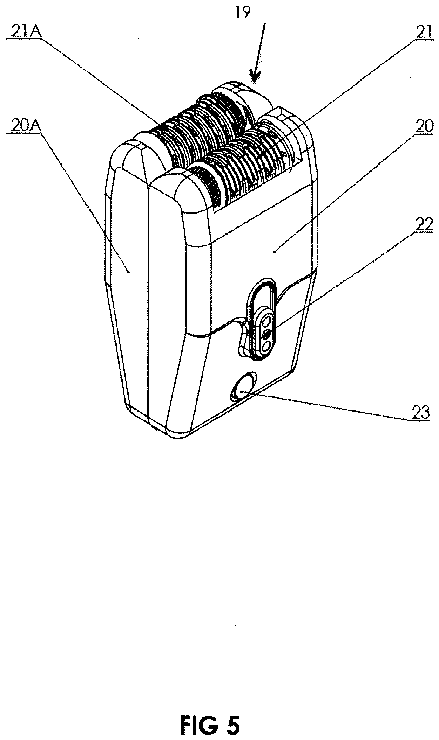

In Fig. 5, fig. 6 and Fig. 7 a further aspect according to the present invention is shown. In these figures the epilator can be separated into two halves while preferably still allowing the epilator to be operated by a user from one side or by one knob or button.

In more detail shows Fig. 5 the two halves of epilator 19 are attached to each other. The heads 21,21A are provided similarly as described before. However, they are either fixedly attached to corresponding driving units 20,20A as shown or are attachable to separate driving units in a releasable manner. In the embodiment shown a switch 22 is provided on one side of one driving unit 20 of the driving units. A release button 23 is preferably provided on the same side as the switch 22.

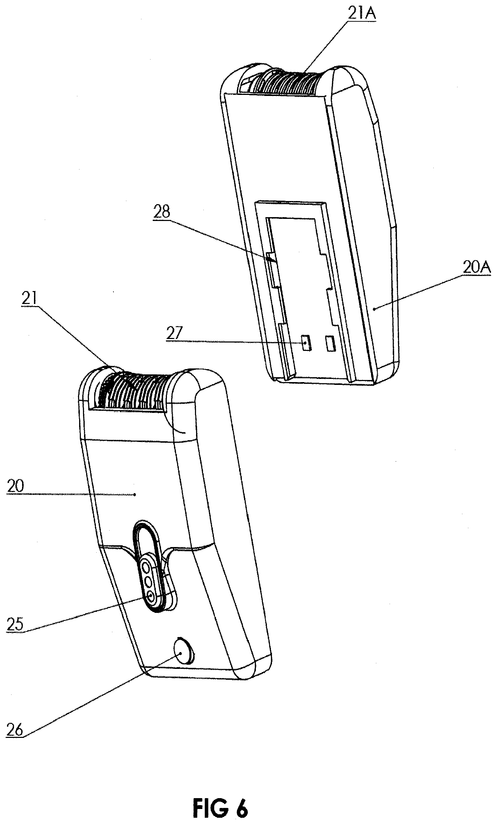

Fig. 6 exemplifies the separation of an epilator according to Fig. 5. It specifically allows a view onto one example of connecting means 28. Slide tracks 28 are shown at the inner side of driving unit 20A engaging corresponding slide tracks (not shown) at the inner side of driving unit 20. In the embodiment shown the drive units 20 and 20A are attached at the inner sides. Electric contacts 27 are also visible which allow a control of both driving units 20,20A by one switch 25. The release button 26 allows the release of both drive units 20,20A upon its activation.

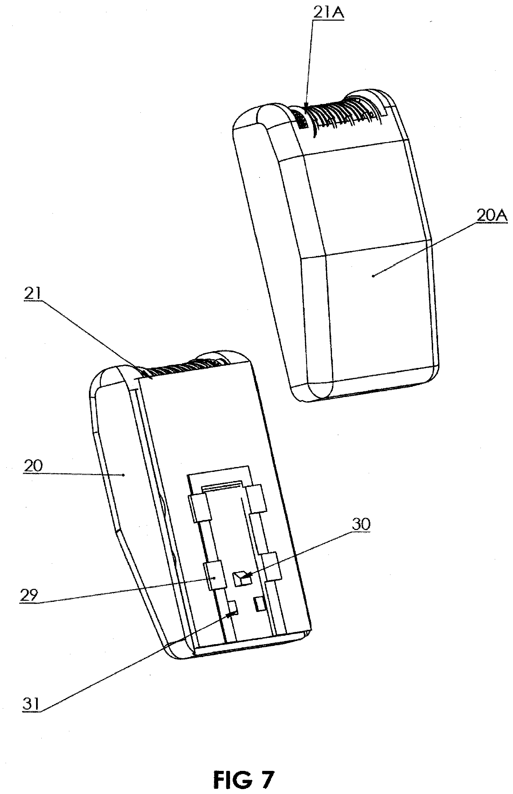

Fig. 6 and Fig. 7 together show one example of the arrangement of both halves of the epilator or of both driving units 20,20A together. Connecting projections 29 arranged at driving unit 20 can be inserted into respective windows or recesses in the driving unit 20A formed into the sliding tracks 28. To be more precise, the upper pair of projections can be inserted into the upper pair of recesses on the opposing side and the lower pair of projections 29 is adapted to be inserted into the lower pair of recesses. The driving units are then moved along their inner surfaces until the projections are positioned in the undercut parts of the sliding tracks 28. Preferably a release button projection 30 arranged in drive unit 20 engages a respective corresponding element (not shown) arranged in drive unit 20A. The two drive units 20,20A snap then into a position where they are aligned with each other. Upon activation of release button 26 on the outer surface of drive unit 20 shown in Fig. 6 the release button projection 30 connected therewith is retracted and allows a repositioning of both driving units 20,20A so that they can be slid again into the position to be taken apart.
Fig. 6 and 7 also show electric contacts 27 at drive unit 20A and corresponding contacts 31 at drive unit 20. They get in touch with each other in the locked position of both drive units 20,20A so that the epilator can be activated by one switch 25 that corresponds to switch 22 shown in Fig. 5.
The embodiments shown and described are intended to exemplify the nature of the present invention and do not limit the scope of the present invention.

## Claims

1. Epilator comprising:
a. a pair of corresponding and separable epilator heads (2,3;21,21A) for trapping hair for removal;
b. each head (2,3;21,21A) having an exposed portion for allowing at least one or more parts of each head (2,3) to get in touch with a user's skin;
c. each head (2,3;21,21A) having a rotating body (17) having an axis of rotation and being adapted to be rotated by a driving unit (1);
d. wherein the rotating body (17) is adapted to trap the hair for removal at the exposed portion; and
e. wherein the axis of the rotating body (17) is essentially concave relative to the exposed portion of each head (2,3).

2. Epilator according to claim 1 wherein each epilator head (2,3; 21,21A) is adapted to be individually coupled to a common driving unit (1) or each epilator head is attached or adapted to be coupled to a corresponding driving unit (20,20A) both of the driving units (20,20A) being adapted to be coupled to and separated from each other.

3. Epilator according to any of the preceding claims, wherein the rotating body (17) of each pair of epilator heads (2,3; 21,21A) are positioned side by side when coupled to the driving unit(s) (1;20,20A).

4. Epilator according to any of the preceding claims, wherein each epilator head (2,3; 21,21A) is coupled to the driving unit (1) in a spring-biased fashion so as to allow a spring-biased movement of each epilator head (2,3) upon pressure thereon during use.

5. Epilator according to any of the preceding claims, wherein the rotating bodies (17) are adapted to be driven by the driving unit(s) (1;20,20A) so as to rotate towards each other in the exposed portions.

6. Epilator according to any of the preceding claims wherein each head (2,3) has a coupling portion (13-16) comprising a coupling element (13a) for a corresponding driving rod (4,5) connected to the driving unit (1), the coupling element (13a) being preferably provided with an open polygonal recess (13) which is further preferably hexagonal so as to allow an axial relative movement of the driving rod (4,5) during driving or use.

7. Epilator according claim 6 wherein the coupling element (13a) is further provided with gear teeth intermeshing with a larger gear (14) driving the rotating body (17) for reducing its rotating speed.

8. Epilator according to any one of the preceding claims wherein each head (2,3) has a snap on mechanism (15,16) for preferably releasable attaching the head (2,3) to the driving unit (1).

9. Epilator according to any one of the preceding claims wherein each head (2,3) further has at least one, preferably a pair of springs (15) biasing each head (2,3) thereby removing any play and allowing a retraction during use of so as to comfortably adapt to the contours of a user upon sufficient pressure.

10. Epilator according to any one of the preceding claims wherein each head (2,3) is adapted to be snapped onto the driving unit (1) of the epilator by a tooth-like snap (10) protruding from the drive unit (1) into a respectively undercut edge (16) or vice versa.

11. Epilator according to any one of the preceding claims wherein the driving units (20,20A) are adapted to be coupled to each other by engaging tracks (28) sliding and engaging in a positively locking manner and are adapted to be released by a release button (23).

12. Epilator according to any of the preceding claims, each head being provided with at least two pincers, the pincers comprising
a. at least one substantially radially extending arm, wherein the arm is provided with a clamping end;
b. an engager which engages the pincers and drives the pincers in use;
c. an actuator driven which causes a relative movement of at least a portion of the pincers so that the pincers are trapping hair in use;
d. at least one spacer located between the two pincers, the spacer being preferably being made of rubber;
e. an axis which receives the pincers, the spacer and/or at least two adjacent discs;
f. the discs further comprising a hub which axially protrudes from the center of a first side of the disc and wherein the hub receives the pincers and the spacer; and
g. at least one first protrusion which substantially axially extends from the first side of the disc;
h. wherein the first protrusion is adapted to cause pincers which are received by the adjacent disc to trap hair during use.

13. Epilator according to any of claims 2 to 12 wherein the drive unit (1) has a driving pin (4,5) for each head (2,3), each driving pin (4,5) extending into a respective coupling element (13) of each head (2,3), each driving pin (4,5) being preferably at its outer end of hexagonal shape corresponding to the one of the respective coupling element (13), and further preferably the driving pins (4,5) and the coupling elements (13) being adapted such that a gap is provided between the ends of the driving pins (4,5) an bottoms of the coupling elements (13).

14. Method of epilating hair, with an epilator according to any one of the preceding claims, comprising the steps of:
a. providing a pair of corresponding and separable epilator heads and trapping hair by the epilator heads for removal;
b. arranging each head with an exposed portion for allowing at least one or more parts of each head to get in touch with a user's skin;
c. arranging and rotating a rotating body in each head, the rotating body having an axis of rotation and being rotated by a driving unit;
d. wherein the rotating body is adapted to trap the hair for removal at the exposed portion; and
e. wherein the axis of the rotating body is arranged to be essentially concave relative to the exposed portion of each head.

15. Method according to claim 14 wherein each epilator head is individually coupled to a common driving unit or each epilator head is attached or coupled to a corresponding driving unit both of the driving units being releasable coupled to each other.

## Patentansprüche

1. Epiliergerät, umfassend:
a. ein Paar entsprechender und trennbarer Epilierköpfe (2,3; 21,21A) zum Erfassen von Haar, um es zu entfernen;
b. wobei jeder Kopf (2,3; 21,21A) einen frei liegenden Abschnitt aufweist, um es mindestens einem oder mehreren Teilen des jeweiligen Kopfes (2,3) zu ermöglichen, mit der Haut eines Benutzers in Kontakt zu kommen;
c. wobei jeder Kopf (2,3; 21,21A) einen rotierenden Körper (17) mit einer Rotationsachse aufweist, der eingerichtet ist, von einer Antriebseinheit (1) gedreht zu werden;
d. wobei der rotierende Körper (17) eingerichtet ist, das Haar am frei liegenden Abschnitt zu erfassen, um es zu entfernen; und
e. wobei die Achse des rotierenden Körpers (17) relativ zum frei liegenden Abschnitt eines jeden Kopfes (2,3) im Wesentlichen konkav ist.

2. Epiliergerät nach Anspruch 1, wobei jeder Epilierkopf (2,3; 21,21A) eingerichtet ist, einzeln mit einer gemeinsamen Antriebseinheit (1) gekoppelt zu werden, oder jeder Epilierkopf befestigt oder eingerichtet ist, um mit einer entsprechenden Antriebseinheit (20,20A) gekoppelt zu werden, wobei beide Antriebseinheiten (20,20A) eingerichtet sind, miteinander gekoppelt und voneinander getrennt zu werden.

3. Epiliergerät nach einem der vorstehenden Ansprüche, wobei die rotierenden Körper (17) von jedem Paar Epilierköpfe (2,3; 21,21A) im an die Antriebseinheit(en) (1; 20,20A) gekoppeltem Zustand nebeneinander angeordnet sind.

4. Epiliergerät nach einem der vorstehenden Ansprüche, wobei jeder Epilierkopf (2,3; 21,21A) mit der Antriebseinheit (1) unter Federbelastung gekoppelt ist, um eine federbelastete Bewegung von jedem Epilierkopf (2,3) bei Drücken auf diesen während des Gebrauchs zu ermöglichen.

5. Epiliergerät nach einem der vorstehenden Ansprüche, wobei die rotierenden Körper (17) eingerichtet sind, durch die Antriebseinheit(en) (1; 20,20A) angetrieben zu werden, um in den frei liegenden Abschnitten zu einander zu rotieren.

6. Epiliergerät nach einem der vorstehenden Ansprüche, wobei jeder Kopf (2,3) einen Kopplungsabschnitt (13-16) hat, der ein Kopplungselement (13a) für eine entsprechende Antriebsstange (4,5) umfasst, die mit der Antriebseinheit (1) verbunden ist, wobei das Kopplungselement (13a) vorzugsweise mit einer offenen polygonalen Vertiefung (13) versehen ist, die weiterhin vorzugsweise sechseckig ist, um eine axiale relative Bewegung der Antriebsstange (4,5) während des Antreibens oder des Gebrauchs zu ermöglichen.

7. Epiliergerät nach Anspruch 6, wobei das Kopplungselement (13a) weiterhin mit Verzahnung bereitgestellt ist, die in ein größeres Zahnrad (14) eingreift, das den rotierenden Körper (17) antreibt, um dessen Rotationsgeschwindigkeit zu reduzieren.

8. Epiliergerät nach einem der vorstehenden Ansprüche, wobei jeder Kopf (2,3) einen Rastmechanismus (15,16) hat, um den Kopf (2,3) vorzugsweise lösbar an der Antriebseinheit (1) zu befestigen.

9. Epiliergerät nach einem der vorstehenden Ansprüche, wobei jeder Kopf (2,3) weiterhin mindestens eine Feder, vorzugsweise ein Paar Federn (15) aufweist, die jeden Kopf (2,3) vorbelasten und somit jegliches Spiel beseitigen und ein Zurückziehen während des Gebrauchs ermöglichen, um eine komfortable Anpassung an die Konturen eines Benutzers bei ausreichendem Druck zu ermöglichen.

10. Epiliergerät nach einem der vorstehenden Ansprüche, wobei jeder Kopf (2,3) eingerichtet ist, auf die Antriebseinheit (1) des Epiliergeräts durch eine zahnartige Rastvorrichtung (10), die von der Antriebseinheit (1) in eine entsprechende hinterschnittene Kante (16) vorsteht oder umgekehrt, aufgerastet zu werden.

11. Epiliergerät nach einem der vorstehenden Ansprüche, wobei die Antriebseinheiten (20,20A) eingerichtet sind, miteinander durch Eingriff in Führungen (28) gekoppelt zu werden, die formschlüssig gleiten und ineinander greifen und eingerichtet sind, durch einen Löseknopf (23) gelöst zu werden.

12. Epiliergerät nach einem der vorstehenden Ansprüche, wobei jeder Kopf mit mindestens zwei Klemmvorrichtungen bereitgestellt ist, wobei die Klemmvorrichtungen umfassen:
a. mindestens einen im Wesentlichen radial verlaufenden Arm, wobei der Arm mit einem klemmenden Ende bereitgestellt ist;
b. ein Eingriffselement, das in die Klemmvorrichtungen eingreift und die Klemmvorrichtungen beim Gebrauch antreibt;
c. einen angetriebenen Aktuator, der eine relative Bewegung von mindestens einem Abschnitt der Klemmvorrichtungen bewirkt, so dass die Klemmvorrichtungen beim Gebrauch Haar erfassen;
d. mindestens einen Abstandshalter, der zwischen den beiden Klemmvorrichtungen angeordnet ist, wobei der Abstandshalter vorzugsweise aus Gummi hergestellt ist;
e. eine Achse, die die Klemmvorrichtungen, den Abstandshalter und/oder mindestens zwei benachbarte Scheiben aufnimmt;
f. wobei die Scheiben weiterhin eine Nabe umfassen, die axial aus der Mitte einer ersten Seite der Scheibe vorsteht und wobei die Nabe die Klemmvorrichtungen und den Abstandshalter aufnimmt; und
g. mindestens einen ersten Vorsprung, der im Wesentlichen von der ersten Seite der Scheibe axial verläuft;
h. wobei der erste Vorsprung eingerichtet ist, zu bewirken, dass Klemmvorrichtungen, die von der benachbarten Scheibe aufgenommen werden, beim Gebrauch Haar erfassen.

13. Epiliergerät nach einem der Ansprüche 2 bis 12, wobei die Antriebseinheit (1) einen Antriebsstift (4,5) für jeden Kopf (2,3) aufweist, wobei jeder Antriebsstift (4,5) in ein jeweiliges Kopplungselement (13) eines jeweiligen Kopfes (2,3) verläuft, wobei jeder Antriebsstift (4,5) vorzugsweise an seinem äußeren Ende entsprechend dem einen der jeweiligen Kopplungselemente (13) sechseckig ausgebildet ist und wobei weiterhin vorzugsweise die Antriebsstifte (4,5) und die Kopplungselemente (13) eingerichtet sind, dass ein Zwischenraum zwischen den Enden der Antriebsstifte (4,5) und Böden der Kopplungselemente (13) bereitgestellt ist.

14. Verfahren zum Epilieren von Haar, mit einem Epiliergerät nach einem der vorstehenden Ansprüche, folgende Schritte umfassend:
a. Bereitstellen eines Paars entsprechender und trennbarer Epilierköpfe und Erfassen von Haar durch die Epilierköpfe, um es zu entfernen;
b. Anordnen eines jeden Kopfes mit einem frei liegenden Abschnitt, um es mindestens einem oder mehreren Teilen des jeweiligen Kopfes zu ermöglichen, mit der Haut eines Benutzers in Kontakt zu kommen;
c. Anordnen und Rotieren eines rotierenden Körpers in jedem Kopf, wobei der rotierende Körper eine Rotationsachse aufweist und durch eine Antriebseinheit rotiert wird;
d. wobei der rotierende Körper eingerichtet ist, das Haar zum Entfernen am frei liegenden Abschnitt zu erfassen; und
e. wobei die Achse des rotierenden Körpers angeordnet ist, um relativ zum freiliegenden Abschnitt eines jeden Kopfes im Wesentlichen konkav zu sein.

15. Verfahren nach Anspruch 14, wobei jeder Epilierkopf einzeln mit einer gemeinsamen Antriebseinheit gekoppelt ist, oder jeder Epilierkopf an einer entsprechenden Antriebseinheit befestigt oder mit ihr gekoppelt ist, wobei beide Antriebseinheiten lösbar miteinander gekoppelt sind.

## Revendications

1. Epilateur comprenant :
a. une paire de têtes d'épilateur correspondantes et séparables (2, 3 ; 21, 21A) pour attraper des poils à enlever ;
b. chaque tête (2, 3 ; 21, 21A) ayant une partie exposée pour permettre à au moins une ou plusieurs parties de chaque tête (2, 3) d'entrer en contact avec la peau d'un utilisateur ;
c. chaque tête (2, 3 ; 21, 21A) ayant un corps rotatif (17) ayant un axe de rotation et étant adapté pour être mis en rotation par une unité d'entraînement (1) ;
d. dans lequel le corps rotatif (17) est adapté pour attraper les poils à enlever au niveau de la partie exposée ; et
e. dans lequel l'axe du corps rotatif (17) est essentiellement concave par rapport à la partie exposée de chaque tête (2, 3).

2. Epilateur selon la revendication 1, dans lequel chaque tête d'épilateur (2, 3 ; 21, 21A) est adaptée pour être couplée individuellement à une unité d'entraînement (1) commune ou chaque tête d'épilateur est fixée ou adaptée pour être couplée à une unité d'entraînement (20, 20A) correspondante, les deux unités d'entraînement (20, 20A) étant adaptées pour être couplées l'une à l'autre et séparées l'une de l'autre.

3. Epilateur selon l'une quelconque des revendications précédentes, dans lequel le corps rotatif (17) de chaque paire de têtes d'épilateur (2, 3 ; 21, 21A) sont positionnés côte à côte lorsqu'ils sont couplés à la ou aux unités d'entraînement (1 ; 20, 20A).

4. Epilateur selon l'une quelconque des revendications précédentes, dans lequel chaque tête d'épilateur (2, 3 ; 21, 21A) est couplée à l'unité d'entraînement (1) par action de ressort de façon à permettre un mouvement dû à l'action d'un ressort de chaque tête d'épilateur (2, 3) en cas de pression sur celle-ci pendant une utilisation.

5. Epilateur selon l'une quelconque des revendications précédentes, dans lequel les corps rotatifs (17) sont adaptés pour être entraînés par la ou les unités d'entraînement (1 ; 20, 20A) de façon à se mettre en rotation l'un vers l'autre dans les parties exposées.

6. Epilateur selon l'une quelconque des revendications précédentes, dans lequel chaque tête (2, 3) a une partie de couplage (13-16) comprenant un élément de couplage (13a) pour une tige d'entraînement (4, 5) correspondante raccordée à l'unité d'entraînement (1), l'élément de couplage (13a) étant de préférence doté d'un évidement polygonal ouvert (13) qui est en outre de préférence hexagonal de façon à permettre un mouvement relatif axial de la tige d'entraînement (4, 5) pendant un entraînement ou une utilisation.

7. Epilateur selon la revendication 6, dans lequel l'élément de couplage (13a) est en outre doté de dents d'engrenage s'engrenant avec un engrenage plus grand (14) entraînant le corps rotatif (17) pour réduire sa vitesse de rotation.

8. Epilateur selon l'une quelconque des revendications précédentes, dans lequel chaque tête (2, 3) a un mécanisme d'encliquetage (15, 16) pour une fixation de préférence libérable de la tête (2, 3) à l'unité d'entraînement (1).

9. Epilateur selon l'une quelconque des revendications précédentes, dans lequel chaque tête (2, 3) a en outre au moins un, de préférence une paire de ressorts (15) agissant sur chaque tête (2, 3), enlevant ainsi tout jeu et permettant une rétractation pendant une utilisation de façon à s'adapter de manière confortable aux contours d'un utilisateur en cas de pression suffisante.

10. Epilateur selon l'une quelconque des revendications précédentes, dans lequel chaque tête (2, 3) est adaptée pour être encliquetée sur l'unité d'entraînement (1) de l'épilateur par un encliquetage de type dent (10) faisant saillie de l'unité d'entraînement (1) dans un bord respectivement évidé (16) ou inversement.

11. Epilateur selon l'une quelconque des revendications précédentes, dans lequel les unités d'entraînement (20, 20A) sont adaptées pour être couplées l'une à l'autre par des glissières de mise en prise (28) coulissant et se mettant en prise de manière solidement verrouillée et sont adaptées pour être libérées par un bouton de libération (23).

12. Epilateur selon l'une quelconque des revendications précédentes, chaque tête étant dotée d'au moins deux pinces, les pinces comprenant
a. au moins un bras s'étendant de manière sensiblement radiale, dans lequel le bras est doté d'une extrémité de serrage ;
b. un organe de mise en prise qui met en prise les pinces et entraîne les pinces pendant une utilisation ;
c. un actionneur entraîné qui amène un mouvement relatif d'au moins une partie des pinces de sorte que les pinces attrapent les poils pendant une utilisation ;
d. au moins un élément d'écartement situé entre les deux pinces, l'élément d'écartement étant de préférence réalisé en caoutchouc ;
e. un axe qui reçoit les pinces, l'élément d'écartement et/ou au moins deux disques adjacents ;
f. les disques comprenant en outre un moyeu qui fait saillie axialement à partir du centre d'un premier côté du disque et dans lequel le moyeu reçoit les pinces et l'élément d'écartement ; et
g. au moins une première saillie qui s'étend de manière sensiblement axiale à partir du premier côté du disque ;
h. dans lequel la première saillie est adaptée pour amener les pinces qui sont reçues par le disque adjacent à attraper les poils pendant une utilisation.

13. Epilateur selon l'une quelconque des revendications 2 à 12, dans lequel l'unité d'entraînement (1) a une broche d'entraînement (4, 5) pour chaque tête (2, 3), chaque broche d'entraînement (4, 5) s'étendant dans un élément de couplage (13) respectif de chaque tête (2, 3), chaque broche d'entraînement (4, 5) étant de préférence au niveau de son extrémité externe de forme hexagonale correspondant à celle de l'élément de couplage (13) respectif, et en outre de préférence les broches d'entraînement (4, 5) et les éléments de couplage (13) étant adaptés de sorte qu'un vide est fourni entre les extrémités des broches d'entraînement (4, 5) et les parties inférieures des éléments de couplage (13).

14. Procédé d'épilation de poils, avec un épilateur selon l'une quelconque des revendications précédentes, comprenant les étapes consistant à :
a. fournir une paire de têtes d'épilateur correspondantes et séparables et attraper des poils par les têtes d'épilateur en vue de les enlever ;
b. agencer chaque tête avec une partie exposée pour permettre à au moins une ou plusieurs parties de chaque tête d'entrer en contact avec la peau d'un utilisateur ;
c. agencer et mettre en rotation un corps rotatif dans chaque tête, le corps rotatif ayant un axe de rotation et étant mis en rotation par une unité d'entraînement ;
d. dans lequel le corps rotatif est adapté pour attraper les poils à enlever au niveau de la partie exposée ; et
e. dans lequel l'axe du corps rotatif est agencé pour être essentiellement concave par rapport à la partie exposée de chaque tête.

15. Procédé selon la revendication 14, dans lequel chaque tête d'épilateur est couplée individuellement à une unité d'entraînement commune ou chaque tête d'épilateur est fixée ou couplée à une unité d'entraînement correspondante, les deux unités d'entraînement étant couplées de manière libérable l'une à l'autre.
